# FASCICULE DE BREVET EUROPEEN

(11) **EP 0 710 240 B1**
(45) Date de publication et mention de la délivrance du brevet: **18.12.1996**
(21) Numéro de dépôt: 94922935.5
(22) Date de dépôt: 19.07.1994
(51) Int. Cl.: C07D 519/04, A61K 31/475

(54) **DERIVES ANTIMITOTIQUES DES ALCALOIDES BINAIRES DU CATHARANTHUS ROSEUS**
ANTIMITOTISCHE DERIVATE VON BINÄREN CATHARANTHUS ROSEUS
NOVEL ANTIMITOTIC BINARY ALKALOID DERIVATIVES EXTRACTED FROM CATHARANTHUS ROSEUS

(30) Priorité: 21.07.1993 FR 9308948
(43) Date de publication de la demande: 08.05.1996
(73) Titulaire: PIERRE FABRE MEDICAMENT, 92100 Boulogne (FR)
(72) Inventeur: JACQUESY, Jean-Claude, F-86180 Buxerolles (FR); FAHY, Jacques, F-81290 Labruguière (FR); BERRIER, Christian, F-86360 Chasseneuil-du-Poitou (FR); BIGG, Dennis, F-81100 Castres (FR); JOUANNETAUD, Marie-Paule, F-86000 Poitiers (FR); ZUNINO, Fabien, F-86000 Poitiers (FR); KRUCZYNSKI, Anna, F-81100 Castres (FR); KISS, Robert, B-1030 Bruxelles (BE)
(74) Mandataire: Ahner, Francis
(86) Numéro de dépôt international: FR9400898
(87) Numéro de publication internationale: WO9503312

(56) Documents cités:
- EP-A- 0 010 458
- US-A- 4 203 898

## Description

Les alcaloïdes antimitotiques extraits de *Catharanthus roseus,* utilisés en chimiothérapie anticancéreuse depuis plus de trente ans, sont représentés principalement par la vinblastine (R = CH₃) et la vincristine (R = CHO).

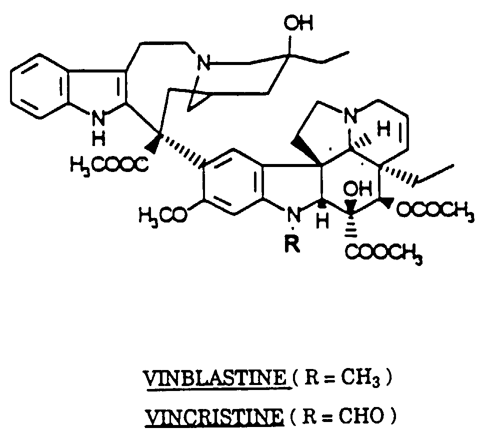

Depuis, de très nombreux dérivés d'hémisynthèse ont été étudiés tant sur le plan chimique que pharmacologique, et quelques uns seulement sont parvenus au stade des études cliniques [O. Van Tellingen, J.H.M. Sips, J.H. Beijnen, *k* Bult et W.J. Nooijen, Anticancer Research, 12, 1699-1716 (1992)].

Seulement deux produits supplémentaires, obtenus par hémi-synthèse, ont été commercialisés à travers le monde: la vindesine par les laboratoires E. Lilly en 1983, et la vinorelbine par les laboratoires Pierre Fabre en 1989.

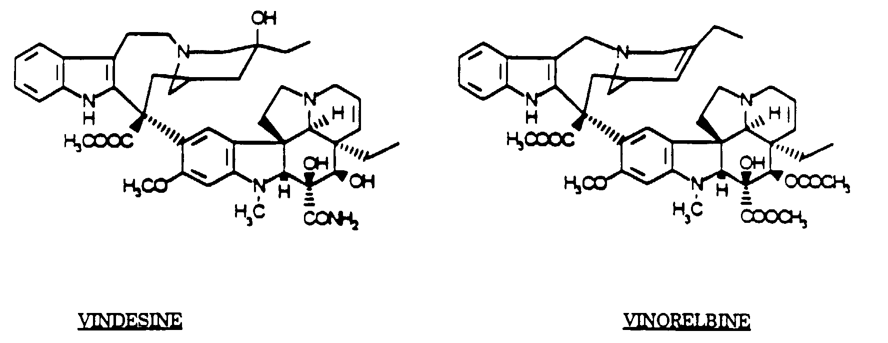

Dans le cadre de notre programme de recherche de nouveaux antimitotiques, nous avons découvert de façon surprenante que les alcaloïdes binaires de la famille de la vinblastine et de la vinorelbine réagissent de façon sélective dans les milieux de type "superacide", pour conduire à de nouveaux produits, fluorés sur des sites inaccessibles par les voies chimiques classiques.

La présente invention a pour objet de nouveaux composés chimiques dérivés des alcaloïdes binaires du *Catharanthus roseus,* leur préparation et leur application en thérapeutique.

Les composés de l'invention possèdent la formule générale 1: 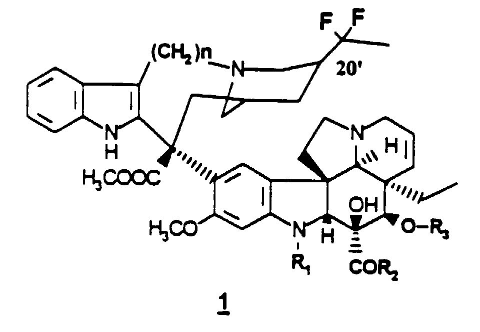 dans laquelle:
n est égal à 1 ou 2,
R₁ représente un groupe méthyle ou un groupe formyle,
R₂ représente un groupe méthoxyle ou un groupe amino,
R₃ représente un atome d'hydrogène ou un groupe acétyle.

L'invention concerne également les sels des composés de formule 1 avec des acides minéraux ou organiques pharmaceutiquement acceptables. L'acide employé peut être, à titre d'exemple non limitatif, l'acide sulfurique ou l'acide tartrique.

L'invention concerne aussi bien les mélanges des diastéréoisomères correspondant aux configurations du carbone 20' des composés de formule générale 1, ainsi que leur mélange en toute proportion.

Les dérivés de l'invention sont préparés par réaction d'un composé de formule générale 2 en milieu superacide, provenant de l'association d'un acide de Bronsted comme l'acide fluorhydrique HF et d'un acide de Lewis comme le pentafluorure d'antimoine SbF₅, en présence d'un réactif halogénant comme le brome, un hypochlorite tel que l'hypochlorite de calcium, ou une N-halogéno imide telle que la N-chlorosuccinimide ou la N-bromosuccinimide.

Les composés de formule générale 2 possèdent la structure décrite ci-dessous: 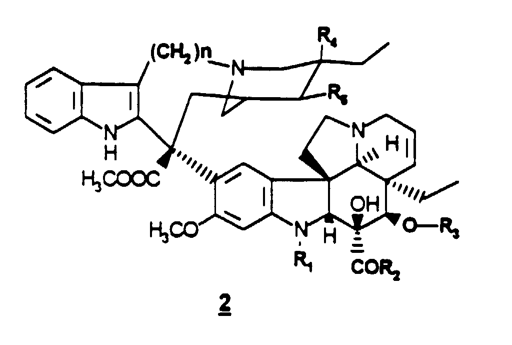 dans laquelle:
n, R₁, R₂ et R₃ sont définis comme ci-dessus,
R₄ représente un groupe hydroxyle et
R₅ représente un atome d'hydrogène; ou bien
R₄ et R₅ forment ensemble une double liaison.

La réaction s'effectue à une température comprise entre -60° C et -15° C dans des récipients en Téflon suivant le schéma suivant:

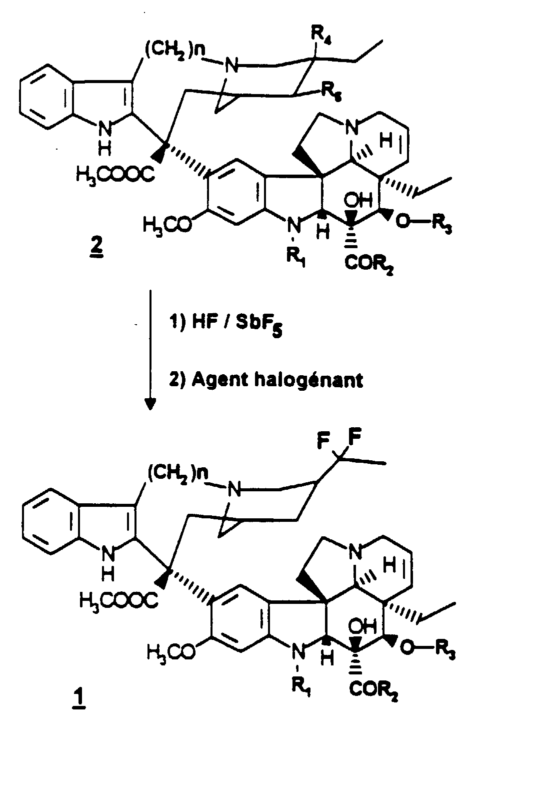

Les composés de formule générale 1 où R₃ = H peuvent également être préparés par hydrolyse de la fonction ester des composés 1 où R₃ = COCH₃. Cette étape est effectuée de préférence dans le méthanol en présence de méthylate de sodium, suivant le schéma suivant: 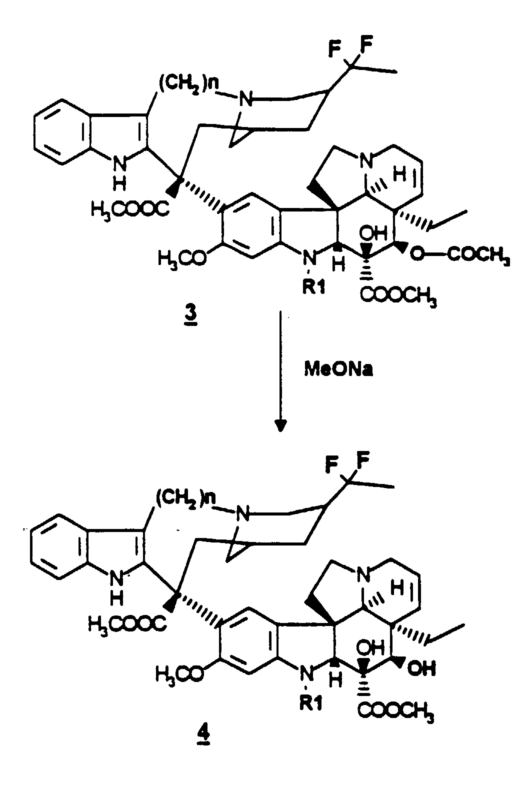 où 3 correspond au composé 1 quand R₃ = COCH₃, et 4 correspond au composé 1 quand R₃ = H.

Les exemples suivants illustrent l'invention, sans toutefois en limiter la portée.

Les caractéristiques spectroscopiques (IR, RMN, masse à haute résolution) confirment la structure des composés obtenus selon l'invention.

Les produits sont décrits à l'aide de la numérotation biogénétique [J. LEMEN et W.I. TAYLOR, Experientia, 21, 508, (1965)].

### Exemple 1:

19',19'-difluoro 15',20'-dihydrovinorelbine 1.
(n = 1, R1 = CH₃, R₂ = OCH₃, R₃ = COCH₃)

A une solution de 33,75 g (156 mmoles) de pentafluorure d'antimoine dans 33,75 g (1690 mmoles) d'acide fluorhydrique anhydre contenue dans un flacon en téflon de 125 ml et refroidie à - 50 °C, on ajoute sous agitation magnétique 2,69 g (2,5 mmoles) de ditartrate de vinorelbine 2 (n = 1, R₁ = CH₃, R₂ = OCH₃, R₃ = COCH₃, R₄ et R₅ = double liaison), puis 0,48 g (2,7 mmoles) de N-bromosuccinimide. L'ensemble est maintenu sous agitation et à - 50 °C pendant 20 minutes.

Le brut réactionnel est ensuite versé rapidement sur un litre de mélange (eau + glace) additionné de 80 g de carbonate de sodium afin d'éviter le réchauffement du mélange. On ajoute ensuite 15 ml d'acétone pour favoriser l'extraction, effectuée par 3 fois 500 ml de dichlorométhane. La phase organique est séparée et séchée sur MgSO₄, et le solvant est évaporé sous pression réduite.

Le résidu obtenu est alors purifié par chromatographie sur colonne de silice éluée par un mélange (CHCl₃: EtOH) dont la composition varie graduellement de (99: 1) à (90: 10). On récupère ainsi 0,52 g (25 %) de 19', 19'-difluoro 15',20'dihydrovinorelbine.

Ce composé est solubilisé dans 3 ml d'EtOH absolu, puis salifié par addition de 1,8 ml de solution à 2 % d'acide sulfurique concentré dans l'EtOH. Le mélange est ensuite versé goutte-à-goutte sous forte agitation dans 20 ml d'éther éthylique refroidi dans un bain de glace. Le précipité blanc obtenu, hygroscopique, est filtré et séché sous vide.
C₄₅ H₅₄ F₂ N₄ O₈, H₂SO₄: 915,01
**Point de fusion:** > 260 °C
IR (KBr): 3437, 2953, 1740, 1618, 1460, 1435, 1234, 1116, 1043 cm-1.
**Spectre de masse à haute résolution** (HRFABMS): pour C₄₅ H₅₅ F₂ N₄ O₈ (MH+):
   - calculé: 817,3987
   - mesuré: 817,3999
**RMN** ^{**1**}**H (200 MHz, CDCl**_{**3**}**) sur la base libre:**
   0,70 (3 H, t, *J* = 7,4 Hz, C₁₈H); 1,18 - 1,45 (4 H, m large); 1,63 (3 H, t, ^{*J*}HF = 18,9 Hz, C₁₈'H); 1,53 - 1,94 (4 H, m large); 2,10 (3 H, s, COCH₃); 2,30 - 2,38 (2 H, m large); 2,55 (1 H, s, C₂₁H); 2,63 - 2,79 (2 H, m large); 2,72 (3 H, s, N-CH₃); 2,90 - 3,12 (2 H, m); 3,18 - 3,40 (4 H, m large); 3,71 (3 H, s, OCH₃); 3,73 (1 H, s, C₂H); 3,79 (3 H, s, OCH₃); 3,82 (3 H, s, OCH₃); 4,45 (1 H, d, *J*= 11,8 Hz, C_{6'}H'); 4,55 (1 H, d, *J* = 11,8 Hz, C_{6'}H'); 5,28 (1 H, d, *J* = 10,2 Hz, C₁₅H); 5,41(1 H, s, C₁₇H); 5,86 (1H, dd, *J* = 10,2/3,8 Hz, C₁₄H); 6,09 (1 H, s, C₁₂H); 6,35 (1 H, s, C₉H); 7,16 (3 H, m, C_{10'}H, C_{11'}H et C_{12'}H); 7,71 (1 H, m, C_{9'}H); 8,42 (1 H, éch., C₁₆OH); 9,86 (1 H, éch., NH).

### Exemple 2:

19',19'-difluoro 15',20'-dihydrovinorelbine 1
(n = 1, R₁ = CH₃, R₂ = OCH₃, R₃ = COCH₃)

Ce dérivé est obtenu suivant le mode opératoire décrit à l'Exemple 1, en remplaçant la N-bromosuccinimide par de la N-chlorosuccinimide lors du traitement en milieu superacide.

Le temps de réaction en milieu superacide est de 20 minutes à - 50 °C.

Les caractéristiques physico-chimiques et spectroscopiques du produit isolé sont identiques à celles du composé obtenu à l'Exemple 1.

### Exemple 3:

19',19'-difluoro 15',20'-dihydrovinorelbine 1
(n = 1, R₁ = CH₃, R2 = OCH₃, R₃ = COCH₃)

Ce dérivé est obtenu suivant le mode opératoire décrit à l'Exemple 1, en ajoutant 0,7 équivalent de brome par rapport à la vinorelbine à la place de la N bromosuccinimide lors du traitement en milieu superacide.

Le temps de réaction en milieu superacide est de 15 minutes à - 30 °C.

Les caractéristiques physico-chimiques et spectroscopiques du produit isolé sont identiques à celles du composé obtenu à l'Exemple 1.

### Exemple 4:

19',19'-difluoro 15',20'-dihydrovinorelbine 1
(n = 1, R₁ = CH₃, R₂ = OCH₃, R₃ = COCH₃)

Ce dérivé est obtenu suivant le mode opératoire décrit à l'Exemple 1, en remplaçant la N-bromosuccinimide par de l'hypochlorite de calcium lors du traitement en milieu superacide.

Le temps de réaction en milieu superacide est de 20 minutes à - 50 °C.

Les caractéristiques physico-chimiques et spectroscopiques du produit isolé sont identiques à celles du composé obtenu à l'Exemple 1.

### Exemple 5:

20'-désoxy 19',19'-difluorovinblastine 1
(n = 2, R₁ = CH₃, R₂ = OCH₃, R₃ = COCH₃)

Ce dérivé est obtenu suivant le mode opératoire décrit à l'**Exemple 1**, en utilisant le sulfate de vinblastine de formule 2 ( n = 2, R₁ = CH₃, R₂ = OCH₃, R₃ = COCH₃, R₄ = OH et R₅ = H) à la place du ditartrate de vinorelbine.

Le temps de réaction en milieu superacide est de 15 minutes à - 30 °C.
C₄₆ H₅₆ F₂ N₄ O₈, H₂SO₄: 929,04
**Point de fusion:** 180 - 186 °C (déc.)
IR (KBr):
   3453, 2957, 1741, 1618, 1512, 1460, 1439, 1371, 1226, 1037, 900 cm⁻¹
**Spectre de masse à haute résolution** (HRFABMS):
   pour C₄₆ H₅₇ F₂ N₄ O₈ (MH+):
   - calculé: 831,4144
   - mesuré: 831,3979
**RMN** ^{**1**}**H (200 MHz, CDCl**_{**3**}**) sur la base libre:**
   0,81 (3 H, t, *J* = 7,4 Hz, Cl₈H); 1,23 - 1,60 (7 H, m large); 1,55 (3 H, t, JHF = 19,2 Hz, C_{18'}H); 2,12 (3 H, s, COCH₃); 2,41 (5 H, m large); 2,66 (1 H, s, C₂₁H); 2,63 - 2,88 (2 H, m large); 2,73 (3 H, s, N-CH₃); 3,22 (8 H, m large); 3,64 (3 H, s, OCH₃); 3,76 (1 H, s, C₂H); 3,80 (3 H, s, OCH₃); 3,82 (3 H, s, OCH₃); 5,32 (1H, d, *J* = 10,2 Hz, C₁₅H); 5,45 (1 H, s, C₁₇H); 5,88 (1 H, dd, *J* = 10,2 / 3,8 Hz, C₁₄H; 6,11 (1H, s, C₁₂H; 6,57 (1H, s, C₉H); 7,16 (3 H, m, C_{10'}H, C_{11'}H et C_{12'}H; 7,49 (1 H, m, C_{9'}H; 8,04 (1 H, éch., C₁₆OH); 9,85 (1 H, éch., NH).

### Exemple 6:

20'-désoxy 19',19'-difluorovinblastine 1
(n = 2, R₁ = CH₃, R₂ = OCH₃, R₃ = COCH₃)

Ce dérivé est obtenu suivant le mode opératoire décrit à l'**Exemple 1**, en utilisant la 15',20'-anhydrovinblastine de formule 2( n = 2, R₁ = CH₃, R₂ = OCH₃, R₃ = COCH₃, R₄ et R₅ = double liaison) à la place du ditartrate de vinorelbine.

Le temps de réaction en milieu superacide est de 15 minutes à - 30 °C.

Les caractéristiques physico-chimiques et spectroscopiques du produit isolé sont identiques à celles du dérivé obtenu à l'**Exemple 5.**

### Exemple 7:

20'-désoxy 19',19'-difluorovindésine 1
(n = 2, R₁ = CH₃, R₂ = NH₂, R₃ = H)

Ce dérivé est obtenu en suivant le mode opératoire décrit à l'**Exemple** 1, en utilisant la vindésine 2(n = 2, R₁ = CH₃, R₂ = NH₂, R₃ = H, R₄ = OH, R₅ = H) à la place du ditartrate de vinorelbine.

Le temps de réaction en milieu superacide est de 15 mn à -30 °C.
C₄₃ H₅₃ F₂ N₅ O₆: 773,92
**Point de fusion:** 186 °C (déc.)
IR (KBr):
   3458, 2924, 2851, 1734, 1686, 1616, 1508, 1458, 1232, 1037 cm-1
**RMN** ^{**1**}**H (200 MHz, CDCl**_{**3**} **+ D**_{**2**}**O):**
   0,94 (3 H, t, *J* = 7,6 Hz, C₁₈H; 1,18 - 1,74 (4 H, m large); 1,55 (3 H, t, ^{*J*} HF = 19,0 Hz, C_{18'}H); 2,07 - 2,55 (4 H, m large); 2,62 (1 H, s, C₂₁H); 2,64 - 2,89 (4 H, m large); 2,90 (3 H,s, N-CH₃); 3,10 - 3,40 (10 H, m large); 3,48 (lH,s, C₂H); 3,62 (3 H, s, OCH₃); 3,80 (3 H, s, OCH₃); 4,14 (1 H, s, C₁₇H); 4,75 (s large, HOD); 5,58 (1 H, d, *J* = 10,8 Hz, C₁₅H); 5,82 (1 H, dd, *J* = 10,8/3,5 Hz, C₁₄H); 6,11 (1 H,s, C₁₂H); 6,55 (1 H,s, C₉H); 7,18 (3 H, m, C_{10'}H, C_{11'}H et C_{12'}H; 7,50 (1 H, d, *J* = 7,1Hz, C_{9'}H).

### Exemple 8:

20'-désoxy 19',19'-difluorovindésine 1
(n = 2, R₁ = CH₃, R₂ = NH₂, R₃ = H)

Ce dérivé est obtenu en suivant le mode opératoire décrit à l'**Exemple 1**, en utilisant la 15',20' -anhydrovindésine 2(n = 2, R₁ = CH₃, R₂ = NH2. R₃ = H, R₄
et R₅ = double liaison) à la place du ditartrate de vinorelbine. Le temps de réaction en milieu superacide est de 15 mn à -30 °C.

Les caractéristiques physico-chimiques et spectroscopiques du produit isolé sont identiques à celles du dérivé ohtenu à l'**Exemple 7**.

### Exemple 9:

Nₐ-desméthyl Nₐ-formyl 19',19'-difluoro 15',20-dihydro-vinorelbine 1
(n = 1, R₁ = CHO, R₂ = OCH₃, R₃ = COCH₃)

Ce dérivé est obtenu suivant le mode opératoire décrit à l'**Exemple 1**, en utilisant le sulfate de Nₐ-desméthyl Nₐ-formyl vinorelbine de formule 2( n = 1, R₁ = CHO, R₂ = OCH₃, R₃ = COCH₃, R₄ et R₅ = double liaison) à la place du ditartrate de vinorelbine.
Le temps de réaction en milieu superacide est de 15 minutes à - 30 °C.
C₄₅ H₅₂ F₂ N₄ O₉: 830,89
**Point de fusion:** > 260 °C (déc.)
IR (KBr):
   3441, 2951, 1745, 1686, 1456, 1371, 1232, 1033, 908,736 cm⁻¹
**RMN** ^{**1**}**H (200 MHz, CDCl**_{**3**}**):**
   0,73 (3 H, t, *J* = 7,5 Hz, C₁₈H): 0,95 - 1,42 (3 H, m large); 1,52 - 1,91 (4 H, m large); 1,62 (3 H, t, ^{*J*}HF = 18,8 Hz, C_{18'}H); 2,06 et 2,09 (3 H, 2s, COCH₃); 2,21 2,29 (2 H, m); 2,59 - 3,11 (6 H, m large); 3,32 (4 H, m); 3,72 (3 H, s, OCH₃); 3,78 et 3,80 (3 H, 2s, OCH₃); 3,91 (3 H, s, OCH₃); 4,42 (1 H, d, *J* = 11,5 Hz, C_{6'}H); 4,52 (1 H, d, *J* = 11,5 Hz, C_{6'}H); 4,49 et 4,72 (1 H, 2s, C₂H); 5,17 et 5,20 (1 H, 2s, C₁₇H); 5,37 (1 H, d, *J* = 10,7 Hz, C₁₅H); 5,88 (1 H, m, C₁₄H); 6,63 et 6,67 (1 H, 2s, C9H); 6,73 et 7,79 (1 H, 2s, C₁₂H); 7,18 (3 H, m, C_{10'}H, C_{11'}H et C_{12'}H); 7,70 (1 H, m, C_{9'}H); 8,43 (1 H, éch., C₁₆OH); 8,16 et 8,75 (1 H, 2s, CHO); 9,35 (1 H, éch., NH).

### Exemple 10:

20'-désoxy 19',19'-difluorovincristine 1
(n = 2, R₁ = CHO, R₂ = OCH₃, R₃ = COCH₃)

Ce dérivé est obtenu suivant le mode opératoire décrit à l'**Exemple 1**, en utilisant le sulfate de vincristine de formule 2 ( n = 2, R₁ = CHO, R₂ = OCH₃, R₃ = COCH₃, R₄ = OH et R₅ = H) à la place du ditartrate de vinorelbine.
Le temps de réaction en milieu superacide est de 15 minutes à - 30 °C.
C₄₆ H₅₄ F₂ N₄ O₉: 844,92
**Point de fusion:** 228 - 233 °C (déc.)
IR (KBr): 3462, 2951, 1743 , 1684, 1597, 1496, 1456, 1369, 1232, 1033 cm-1.
**RMN** ^{**1**}**H (200 MHz, CDCl**_{**3**}**):**
   0,84 (3 H, t, *J* = 7,5 Hz, C₁₈H)1 1,20 - 1,77 (6 H, m large); 1,53 (3 H, t, ^{*J*}HF = 19,0 Hz, C_{18'}H); 2,05 - 2,59 (6 H, m large); 2,07 et 2,09 (3 H, 2s, COCH₃); 2,74 2,92 (1 H, m large); 2,89 (1 H,s, C₂₁H; 3,10 - 3,53 (5 H, m large); 3,69 (3 H, s, OCH₃); 3,72 et 3,79 (3 H, 2s, OCH₃); 3,90 (3 H,s, OCH₃); 4,51 et 4,74 (1 H, 2s, C₂H); 5,21 et 5,25 (1 H , 2s, C₁₇H); 5,41 (1 H, d, *J* = 10,2 Hz, C₁₅H); 5,93 (1 H, dd, *J* = 10,2 / 3,8 Hz, C₁₄H); 6,81 et 6,85 (1 H, 2s, C₉H); 6,90 et 7,76 (1 H, 2s, C₁₂H); 7,18 (3 H, m, C_{10'}H, C_{11'}H et C_{12'}H); 7,52 (1 H, d, *J* = 7,0 Hz, C_{9'}H); 8,07 (1 H, éch., C₁₆OH); 8,17 et 8,77 (1 H, 2s, CHO); 9,70 (1 H, éch., NH).

### Exemple 11:

17-désacétyl 19',19'-difluoro 15',20'-dihydrovinorelbine 1
(n = 1, R₁ = CH₃, R₂ = OCH₃, R₃ = H)

A une solution de 200 mg (0,24 mmoles) de 19',19'-difluoro 15' ,20'-dihydrovinorelbine 1 (n = 1, R₁ = CH₃, R₂ = OCH₃, R₃ = COCH₃), obtenue à **l'Exemple 1**, dans 10 ml de méthanol sec sous atmosphère d' azote et sous agitation, on ajoute 130 mg (2,40 mmoles) de méthylate de sodium. Après 12 heures, le mélange est versé dans 100 ml d'eau + glace, puis extrait par 3 fois 20 ml de dichlorométhane. La phase organique est séparée, séchée sur MgSO₄, filtrée et évaporée. Le résidu obtenu est purifié par chromatographie sur colonne de silice éluée par un mélange Hexane: AcOEt: MeOH (4: 2: 1). On récupère ainsi 134 mg (72 %) de 17-désacétyl 19',19'-difluoro 15',20'-dihydrovinorelbine sous forme de poudre blanchâtre.
C₄₃ H₅₂ F2 N₄ O₇: 774,90
**Point de fusion:** 240 - 245 °C (déc.)
IR (KBr):
   3445, 2947, 1736, 1616, 1504, 1460, 1433, 1234, 1049, 904, 742 cm-1
**Spectre de masse à haute résolution** (HRFABMS):
   pour C₄₃ H₅₃ F₂ N₄ O₇ (MH+)
   - calculé: 775,3882
   - mesuré: 775,3759
**RMN** ^{**1**}**H (200 l\lIHz, CDCl3):**
   0,86 (3 H, t, *J* = 7,4 Hz, C₁₈H); 0,96 - 1,42 (4 H, m large); 1,63 (3 H, t, ^{*J*}HF = 19,0 Hz, C_{18'}H); 1,53 - 2,05 (4 H, m large); 2,05 - 3,48 (12 H, m large); 2,50 (1 H, s, C₂₁H); 2,77 (3 H, s, N-CH₃); 3,70 (3 H, s, OCH₃); 3,81 (3 H, s, OCH₃); 3,84 (3 H, s, OCH₃); 4,01 (1 H, s, C₁₇H); 4,51(2 H, s large, C_{6'}H); 5,70 (1 H, d, *J* = 10,6 Hz, C₁₅H); 5,85 (1 H, dd, *J* = 10,6 / 3,8 Hz, C₁₄H); 6,10 (1 H, s, C₁₂H); 6,35 (1 H, s, C₉H); 7,18 (3 H, m, C_{10'}H, C_{11'}H et C_{12'}H); 7,72 (1 H, m, C_{9'}H); 8,42 (1H , éch., C₁₆OH); 9,50 (1 H, éch., NH).

### Exemple 12:

17-désacétyl 20'-désoxy 19',19'-difluoroviriblastine 1
(n = 2, R₁ = CH₃, R₂ = OCH₃, R₃ = H)

Ce dérivé est obtenu suivant le mode opératoire décrit à l' **Exemple 11**, en remplaçant la 19',19'-difluoro 15',20'-dihydrovinorelbine 1 (n = 1, R₁ = CH₃, R₂ = OCH₃, R₃ = COCH₃) par la 20'-désoxy 19',19'-difluorovinblastine 1 (n = 2, R₁ = CH₃, R₂ = OCH₃, R₃ = COCH₃) obtenue à **l'Exemple 5**. Après purification, la 17-désacétyl 20'-désoxy 19',19'-difluorovinblastine 1 (n = 2, R₁ = CH₃, R₂ = OCH₃, R₃ = H) est salifiée par addition d'un équivalent d'acide tartrique.
C₄₄ H₅₄ F₂ N₄ O₇, C₄H₆O₆: 939,02
**Point de fusion:** 180 - 185 °C (déc.)
IR (KBr):
   3447, 2968, 1734, 1616, 1506, 1460, 1234, 1122, 744 cm-1
**Spectre de masse à haute résolution** (HRFABMS): pour C₄₄ H₅₅ F₂ N₄ O₇(MH+):
   - calculé: 789,4038
   - mesuré: 789,4022
**RMN** ^{**1**}**H (200 MHz, CDCl**_{**3**}**):**
   0,95 (3 H, t, *J* = 7,4 Hz, C₁₈H); 1,18 - 1,74 (4 H, m large); 1,53 (3 H, t, ^{*J*}HF = 19,0 Hz, C_{18'}H); 2,07 - 2,55 (4 H, m large); 2,63 (1 H, s, C₂₁H); 2,64 - 2,89 (4 H, m large); 2,77 (3 H, s, N-CH₃); 3,10 - 3,52 (11 H, m large); 3,62 (3 H, s, OCH₃); 3,72 (1 H, s, C₂H; 3,81 (3 H, s, OCH₃); 3,85 (3 H, s, OCH₃); 4,07 (1 H, s, C₁₇H); 5,75 (1 H, d, *J* = 10,8 Hz, C₁₅H); 5,86 (1 H, dd, *J* = 10,8 / 3,5 Hz, C₁₄H); 6,11 (1 H, s, C₁₂H); 6,61 (1 H, s, C₉H); 7,12 (3 H, m, C_{10'}H, C_{11'}H et C_{12'}H); 7,51 (1H, d, *J* = 7,1 Hz, C_{9'}H); 8,01(1 H, éch., C₁₆OH); 9,30 (1 H, éch., NH).

Les composés de l'invention ont été soumis à des essais pharmacologiques qui ont mis en évidence leur intérêt comme substances à activité thérapeutiques. Aussi, l'activité cytotoxique des produits a été évaluée utilisant le test MTT [T. Mosman, J. Immunol. Method, 65, 55 (1983)] sur différentes lignées cellulaires tumorales. Le test MTT est basé sur la capacité qu'ont les cellules vivantes de réduire, par l'action de leurs enzymes mitochondriales, un sel de tétrazolium jaune en un composé bleu violet, le formazan, mesurable par spectrophotométrie après dissolution dans le diméthylsulfoxide. La quantité de formazan formée (et par conséquent l'intensité de la coloration obtenue) est directement proportionnelle au nombre de cellules vivantes présentes dans le milieu de culture au moment de l'essai. Les lignées utilisées sont d'origine humaine, et sont commercialisées par l'American Type Cell Collection (ATCC), organisme de référence pour la fourniture de souches standardisées.

| **Lignée** | **code ATCC** | **Tumeur d'origine** |
|---|---|---|
| MCF-7 | HTB22 | sein |
| T47D | HTB133 | sein |
| J82 | HTB1 | vessie |
| T24 | HTB4 | vessie |

Le protocole expérimental utilisé est essentiellement celui décrit par C. Carmichael, W.G. De Graff, A.F. Gazdor, D. Minna, et B. Mitchell [Cancer Res., 47, 936 (1987)].

Les résultats sont exprimés sous forme d'un pourcentage d'inhibition de croissance par rapport aux contrôles.

Le tableau 1 donne, à titre d'exemple, les résultats obtenus pour certains dérivés de l'invention à la concentration de 1 µg/ml.

**Tableau 1**

| **Produit de l'exemple** | **Pourcentage d'inhibition de croissance** | | | |
|---|---|---|---|---|
| | **MCF-7** | **T47** | **J82** | **T24** |
| **1** | 68 | 44 | 45 | 70 |
| **5** | 36 | 27 | 15 | 50 |
| **11** | 45 | 30 | 60 | 51 |
| **12** | 36 | 27 | 0 | 72 |
| **VINORELBINE** | 50 | 16 | 14 | 62 |

Comme les alcaloïdes antitumoraux du *Catharanthus roseus,* les produits préparés suivant l'invention sont des poisons du fuseau mitotique.
Cette propriété a été confirmée par la mesure de l'inhibition de polymérisation de la tubuline en microtubules en présence des composés de l'invention, en suivant la méthode décrite par R.C. Weisenberg (Science 177, 1196-7, 1972). Les résultats sont exprimés en concentration de produit qui provoque 50 % d'inhibition de la polymérisation. Ce phénomène est aisément suivi et quantifié par l'intermédiaire des variations de densité optique.

A titre d'exemple, le tableau 2 montre les résultats obtenus avec quelques dérivés préparés suivant l'invention:

| **Produit de l'exemple** | **IC**_{**50**} **(µM)** |
|---|---|
| **1** | 2,50 |
| **5** | 8,9 |
| **11** | 2,9 |
| **12** | 4,2 |
| **VINBLASTINE** | 1,9 |

Les propriétés antitumorales des produits de l'invention ont été confirmées par des tests in vivo, notamment sur le modèle d'adénocarcinome mammaire MXT, tumeur solide particulièrement peu sensible aux agents anticancéreux [C.S Watson, D. Medina, J.H. Clark, Cancer Res., 37, 3344 (1977); W.T. Bradner et C.A. Claridge, "Antineoplastic Agents", Wiley-Interscience (1984); T.W. Redding et A.V. Schally, Proc. Natl. Acad. Sci. U.S.A., 80, 1459 (1983)].

Dans ce modèle, des souris de type B6D2Fl sont greffées par l'injection (sous-cutanée) d'un fragment tumoral d'environ 10 mm³ provenant d'une tumeur MXT. Les produits à tester sont administrés par voie i.p. à partir du 17ème jour après la greffe. Le test MXT fournit deux types de résultats: l'effet exercé par la molécule étudiée sur la croissance tumorale, et le temps de survie des animaux traités par rapport aux animaux contrôles (T/C exprimé en pourcentage).

A titre d'exemple, le composé de l'**Exemple 1** provoque une réduction de taille tumorale de 40 % par rapport aux témoins non traités suivant un protocole de 9 x 40 mg/kg, alors que tous les dérivés de cette famille chimique, vinblastine, vincristine, vindesine et vinorelbine, sont dépourvus d'activité sur ce modèle.

Compte tenu de leurs propriétés pharmacologiques, les composés de la présente invention peuvent être utilisés en thérapeutique humaine dans le traitement de la pathologie cancéreuse.

Les préparations pharmaceutiques contenant ces principes actifs peuvent être mises en forme pour l'administration par voie orale, intraveineuse ou sous-cutanée.

## Revendications

1. Dérivés antimitotiques des alcaloides binaires du *Catharanthus roseus* correspondant à la formule générale 1: 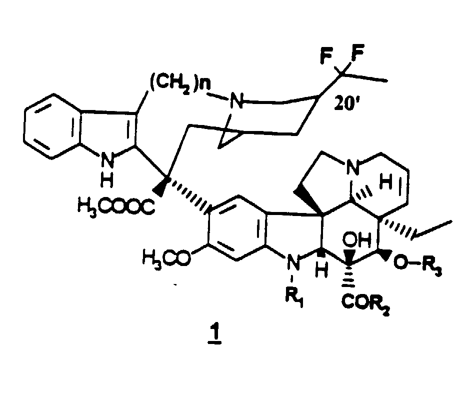 dans laquelle:
n est égal à 1 ou 2,
R₁ représente un groupe méthyle ou un groupe formyle,
R₂ représente un groupe méthoxyle ou un groupe amino,
R₃ représente un atome d'hydrogène ou un groupe acétyle,
ainsi que leurs sels avec des acides minéraux ou organiques thérapeutiquement acceptables, et les mélanges des diastéréoisomères correspondant aux deux configurations du carbone 20' des composés et leurs mélanges en toutes proportions.

2. Composés de formule générale 1 selon la revendication 1, caractérisés par le fait qu'ils sont choisis parmi:
- 19',19'-difluoro 15',20'-dihydrovinorelbine
- 20'-désoxy 19',19'-difluorovinblastine
- 20'-désoxy 19',19'-difiuorovindésine
- Na-desméthyl Na-formyl 19',19'-difluoro 15',20'-dihydrovinorelbine
- 20'-désoxy 19',19'-difluorovincristine
- 17-désacétyl 19',19'-difiuoro 15',20'-dihydro-vinorelbine
- 17-désacétyl 20'-désoxy 19',19'-difiuorovinblastine

3. Procédé de préparation de composés selon les revendications 1 et 2, caractérisé en ce que l'on fait réagir un composé de formule générale 2 en milieu super acide, provenant de l'association d'un acide de Bronsted comme l'acide fluorhydrique HF et d'un acide de Lewis comme le pentafluorure d'antimoine SbFs, en présence d'un agent halogénant selon le schéma:

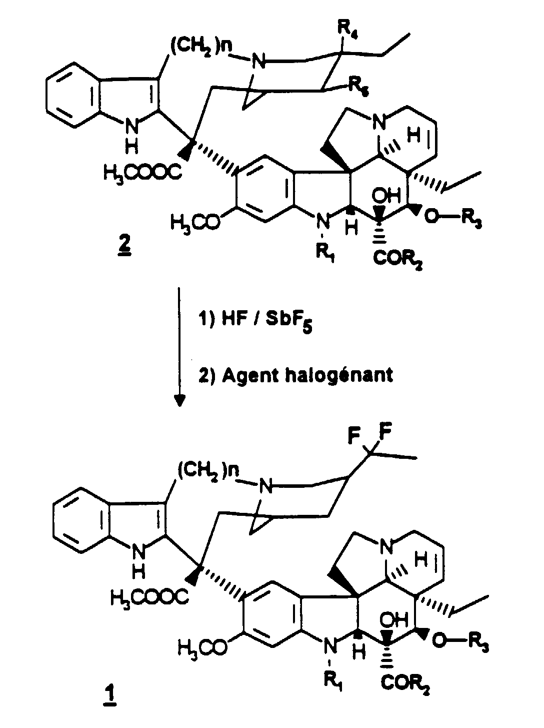

4. Procédé de préparation de composés selon la revendication 3, caractérisé en ce que la réaction d'un composé de formule générale 2 en milieu superacide s'effectue à une température comprise entre -60°C et -15°C.

5. Procédé de préparation de composés selon la revendication 3, caractérisé en ce que l'agent halogénant est choisi parmi:
- le brome,
- l'hypochlorite de calcium,
- la N-chlorosuccinimide,
- la N-bromosuccinimide.

6. Procédé de préparation de composés de formule générale 1 où R₃ représente un atome d'hydrogène, selon les revendications 1 et 2, caractérisé en ce que l'on hydrolyse un composé de formule générale 1 où R₃ représente un groupe acétyle selon le schéma: 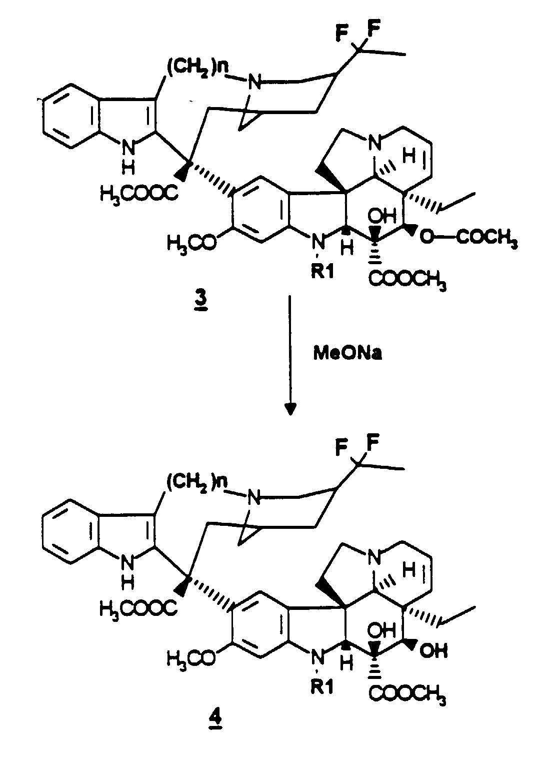
où 3 correspond au composé 1 quand R₃ = COCH₃,
et 4 correspond au composé 1 quand R₃ = H.

7. Procédé de préparation de composés selon la revendication 6, caractérisé en ce que l'hydrolyse s'effectue à l'aide du méthylate de sodium dans le méthanol.

8. A titre de médicaments nouveaux utiles, par exemple, en thérapeutique humaine dans le traitement de la pathologie cancéreuse, les composés définis selon l'une des revendications 1 et 2.

9. Composition pharmaceutique caractérisée en ce qu'elle contient à titre de principe actif au moins un composé selon l'une des revendications 1 et 2, associé à un véhicule pharmaceutique acceptable.

## Patentansprüche

1. Antimitotische Derivate von binären Alkaloiden aus Catharanthus roseus, entsprechend der allgemeinen Formel 1: 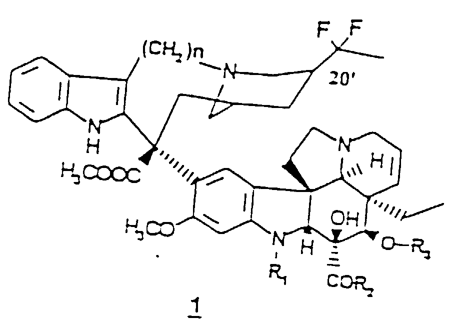 wobei
n 1 oder 2 ist,
R₁ eine Methylgruppe oder Formylgruppe darstellt,
R₂ eine Methoxygruppe oder Aminogruppe darstellt,
R₃ ein Wasserstoffatom oder eine Acetylgruppe darstelt,
sowie therapeutisch verträglichen Salze von Mineralsäuren oder organischen Säuren, und ihren Diasteromer-Mischungen,
entsprechend den beiden Konfigurationen des Kohlenstoffatoms 20' der Verbindungen und ihren Gemischen in jedem Verhältnis.

2. Verbindungen der allgemeinen Formel 1 nach Anspruch 1, dadurch charakterisiert, daß sie ausgewählt werden aus:
- 19',19'-Difluoro-15',20'-dihydrovinorelbin
- 20'-Desoxy-19',19'-difluorovinblastin
- 20'-Desoxy-19',19'-difluorovindesin
- Na-Desmethyl-Na-formyl-19',19'-difluoro-15',20'-dihydrovinorelbin
- 20'-Desoxy-19',19'-difluorovincristin
- 17'-Desacetyl-19',19'-difluoro-15',20'-dihydrovinorelbin
- 17-Desacetyl-20'-desoxy-19',19'-difluorovinblastin

3. Herstellungsverfahren für Verbindungen nach den Ansprüchen 1 und 2, dadurch charakterisiert, daß man eine Verbindung der allgemeinen Formel 2 in supersaurem Milieu aus einer Verbindung aus einer Brönsted-Säure wie Fluorwasserstoffsäure HF und einer Lewis-Säure wie Antimonpentafluorid SbFS in Gegenwart eines Halogenierungsmittels nach folgendem Schema umsetzt:

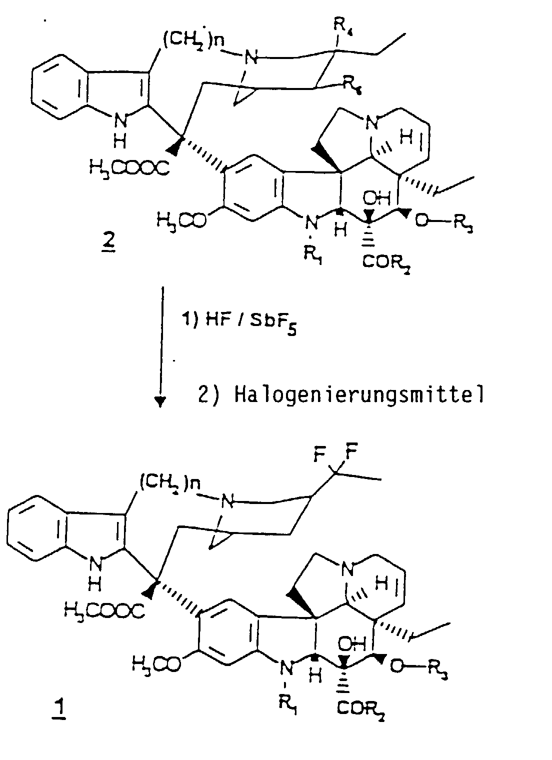

4. Herstellungsverfahren für Verbindungen nach Anspruch 3, dadurch charakterisiert, daß die Reaktion einer Verbindung der allgemeinen Formel 2 in supersaurem Milieu bei einer Temperatur zwischen -60°C und -15°C durchgeführt wird.

5. Herstellungsverfahren für Verbindungen nach Anspruch 3, dadurch charakterisiert, daß das Halogenierungsmittel ausgewählt wird aus:
- Brom,
- Calciumhypochlorit,
- N-Chlor-succinimid,
- N-Brom-succinimid.

6. Herstellungsverfahren für Verbindungen der allgemeinen Formel 1, wobei R₃ ein Wasserstoffatom darstellt, entsprechend den Ansprüchen 1 und 2, dadurch charakterisiert, daß man eine Verbindung der allgemeinen Formel 1, wobei R₃ eine Acetylgruppe darstellt, nach dem folgenden Schema hydrolysiert: 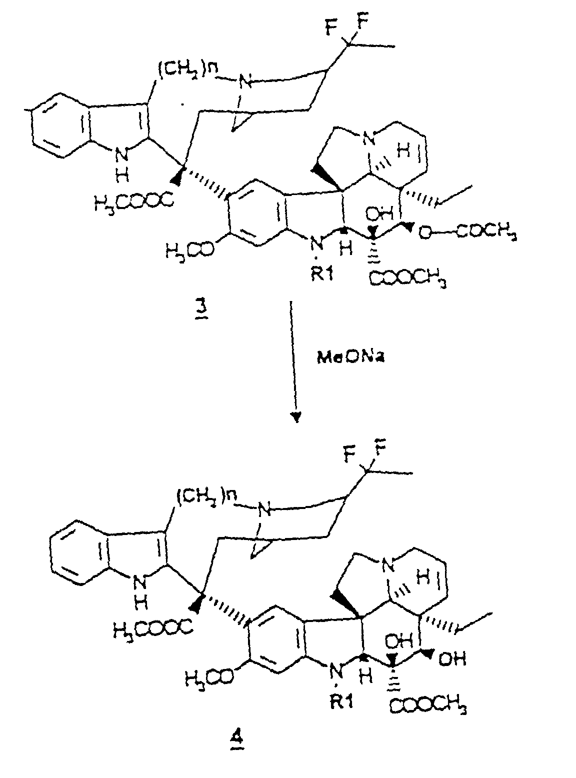 wobei 3 der Verbindung 1 entspricht, wenn R₃ = COCH₃, und 4 der Verbindung 1 entspricht, wenn R₃ = H.

7. Herstellungsverfahren für Verbindungen nach Anspruch 6, dadurch charakterisiert, daß die Hydrolyse mit Hilfe von Natriummethylat in Methanol durchgeführt wird.

8. Verbindungen nach einem der Ansprüche 1 und 2 zur Verwendung als neue nützliche Medikamente, beispielsweise bei der Therapie des Menschen in der Behandlung von Krebs.

9. Pharmazeutische Zusammensetzung, dadurch charakterisiert, daß sie als aktiven Wirkstoff mindestens eine Verbindung nach einem der Ansprüche 1 und 2 in Verbindung mit einem pharmazeutisch verträglichen Träger enthält.

## Claims

1. Antimitotic derivatives of binary alkaloids from *Catharanthus roseus* corresponding to the general formula 1: 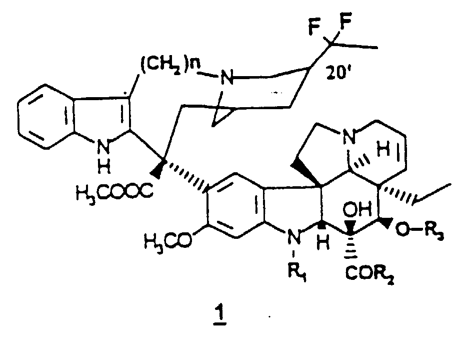 in which:
n is equal to 1 or 2,
R₁ represents a methyl group or a formyl group,
R₂ represents a methoxy group or an amino group,
R₃ represents a hydrogen atom or an acetyl group,
as well as the salts thereof with therapeutically acceptable inorganic or organic acids, and the mixtures of the diastereoisomers corresponding to the two configurations of carbon 20' of the compounds and their mixtures in all proportions.

2. Compounds of the general formula 1 according to Claim 1, characterized in that they are chosen from:
- 19',19'-difluoro 15',20'-dihydrovinorelbine
- 20'-deoxy 19',19'-difluorovinblastine
- 20'-deoxy 19',19'-difluorovindesine
- Na-demethyl Na-formyl 19',19'-difluoro 15',20'-dihydrovinorelbine
- 20'-deoxy 19',19'-difluorovincristine
- 17-deacetyl 19',19'-difluoro 15',20'-dihydrovinorelbine
- 17-deacetyl 20'-deoxy 19',19'-difluorovinblastine

3. Process for the preparation of compounds according to Claims 1 and 2, characterized in that a compound of general formula 2 is reacted in superacid medium, originating from the combination of a Bronsted acid such as hydrofluoric acid HF and a Lewis acid such as antimony pentafluoride SbF₅, in the presence of a halogenating agent, according to the scheme:

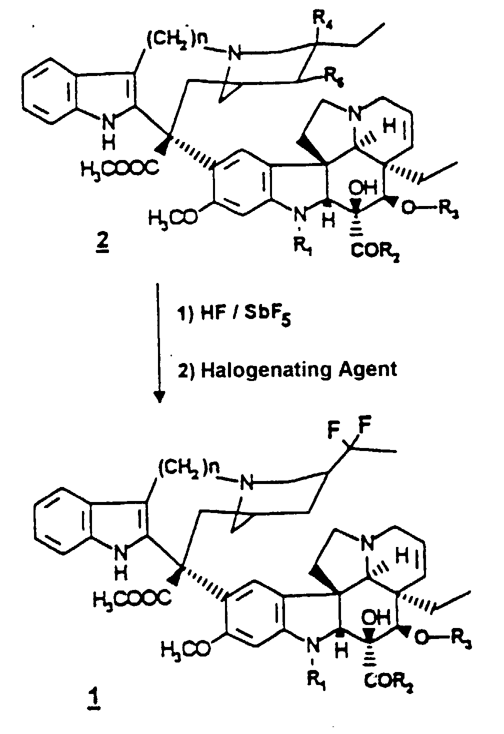

4. Process for the preparation of compounds according to Claim 3, characterized in that the reaction of a compound of general formula 2 in superacid medium is carried out at a temperature between -60°C and -15°C.

5. Process for the preparation of compounds according to Claim 3, characterized in that the halogenating agent is chosen from:
- bromine,
- calcium hypochlorite,
- N-chlorosuccinimide,
- N-bromosuccinimide.

6. Process for the preparation of compounds of general formula 1 where R₃ represents a hydrogen atom, according to Claims 1 and 2, characterized in that a compound of general formula 1, where R₃ represents an acetyl group, is hydrolysed according to the scheme: 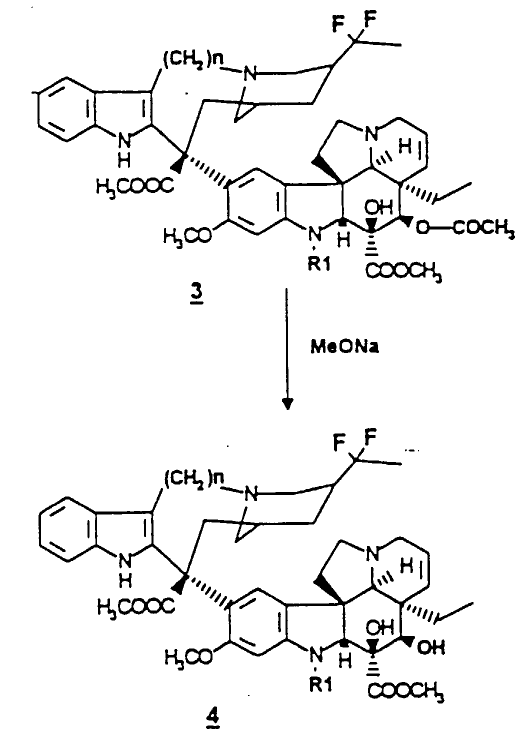 where 3 corresponds to the compound 1 when R₃ = COCH₃, and 4 corresponds to the compound 1 when R₃ = H.

7. Process for the preparation of compounds according to Claim 6, characterized in that the hydrolysis is carried out using sodium methoxide in methanol.

8. By way of novel medicinal products which are useful, for example, in human therapy in the treatment of cancer pathology, the compounds defined according to either of Claims 1 and 2.

9. Pharmaceutical composition, characterized in that it contains, as active principle, at least one compound according to either of Claims 1 and 2, combined with a pharmaceutically acceptable vehicle.
